# EUROPEAN PATENT SPECIFICATION

(11) **EP 2 279 769 B1**
(45) Date of publication and mention of the grant of the patent: **07.05.2014**
(21) Application number: 09741714.1
(22) Date of filing: 06.05.2009
(51) Int. Cl.: A61M 5/178, A61M 5/50

(54) **A DISPOSABLE SELF-DESTRUCTION SAFETY SYRINGE WITHOUT FLUID RESIDUA**
SELBSTZERSTÖRENDE SICHERHEITSSPRITZE ZUM EINMALIGEN GEBRAUCH OHNE FLÜSSIGKEITSRÜCKSTÄNDE
SERINGUE DE SECURITE A AUTODESTRUCTION A USAGE UNIQUE SANS FLUIDE RESIDUEL

(30) Priority: 08.05.2008 CN 200810096785; 13.04.2009 CN 200920150008 U
(43) Date of publication of application: 02.02.2011
(73) Proprietor: Shantou Wealy Medical Instrument Co., Ltd, Guangdong 515064 (CN)
(72) Inventor: YANG, Yuhe, Shantou, Guangdong 515000 (CN)
(74) Representative: Vossius & Partner
(86) International application number: PCT/CN2009/071657
(87) International publication number: WO 2009/135439

(56) References cited:
- EP-A1- 1 625 865
- WO-A1-2006/119570
- WO-A1-2006/119570
- WO-A1-2007/019647
- WO-A2-2006/111806
- CN-A- 1 252 311
- CN-A- 1 509 194
- CN-Y- 2 778 297
- US-A1- 2005 096 604

## Description

### Field of the Invention

The present invention relates to a self-destruction safety syringe, in particular, to a disposable self-destruction safety syringe without fluid residua.

### Background of the Invention

Syringe is one of the most common medical devices. In order to reduce the spread of the blood-borne diseases among the patients, all the countries in the world, including our country, make law to compulsorily popularize and use the disposable syringe. However, current common disposable syringes have defects in that they generally might be repeatedly used and are not provided with safety device which causes the needle to be exposed etc. Therefore, on one hand, the disposable syringes are easily used by the lawbreakers or drug addicts; and on the other hand, the syringes that are supposed to be disposable are reused, which easily causes the virus spread and cross infection. In order to prevent the phenomena that the disposable syringes are re-purchased, re-sold and reused and to avoid the infection of the medical care personnel whom might be stuck by the syringe needle when collecting and processing the syringes, all the countries in the world are under the research of the self-destruction safety syringe and popularizing the application of it. Self-destruction safety syringes currently known are mainly of the type of piercing plunger or manual pull-type with syringe needle, and there is also another type that the needle mount is provided with an automatic spring, after the fluid has been injected, the push rod is pushed to the frontmost end of the syringe tube, meanwhile, the needle mount is retracted together with the syringe needle into the syringe tube to prevent the protruding syringe needle from sticking people. But all these self-destruction safety syringes still have various defects in designs.

A Chinese patent with a grant announcement number of CN200980874Y discloses a safety self-destruction syringe, in which the needle mount and the syringe needle can automatically retract into the push tube by providing an automatic retraction means in the push tube. But this invention neither thoroughly solves the problem of how to compress the compression spring to the lower part of the push tube before use (the inside wall of the push tube is smooth without any object for fastening and the compression spring cannot be compressively fastened) nor solves the problem of how the residual fluid passes through in the interference fit.

A US patent application with publishing number Of US5211628A discloses a syringe with automatic retracting means. This syringe uses a biasing member of spring to encircling the shank portion of the connecting shaft; the spring has an upper end engaged with the top terminal wall of the push rod and a lower end embedded and locked in the brake groove by the biasing member; in addition, the actuating section has a pushing terminal moving together with the puncturing end of the connecting shaft. There is a pulling terminal, when the coupling end of the connecting shaft disengages from the outer ring contiguous area under pressure, the pushing terminal on the spring will move downwardly together with the puncturing end, the pulling terminal can pull the lower end of the spring out of the brake groove; moreover, a gasket is placed to bring the bulge ring to the rear chamber of the outer cover to be engaged with the front wall of the rear chamber, to provide a resistance for the formation of the gasket. This resistance supports positioning of the bulge ring to prevent movement of a certain position of the front wall of the back chamber of the gasket in hypodermic or intravenous injection puncture, thereby the bulge ring being allowed to disengage from a certain portion of the back chamber of the syringe, therefore, when the first external force is exerted, the tubular needle gauge can move with the bulge ring in the inside wall of the outer cover. However, this syringe still has some defects: when the smallest part of the snap-latch means and the biggest part of the needle mount form interference fit with each other, the residual fluid is sealed and cannot circulate, at this moment, if the push rod is pushed forward with a bigger pressure, the needle punctured subcutaneously will be vibrated and causes pain to the patient given the injection.

The Chinese Patent with a grant announcement number of CN2817873Y also discloses a self-destruction safety syringe. This syringe uses a spring-back device of a steel needle which is arranged in the outer needle mount to form a needle head assembly connected with the top end of the injection cylinder through threads. This syringe not only has the characteristic of the ordinary disposable syringe with the spring-back steel needle, but also the producing process thereof becomes simple and the assembly is convenient, etc. However, it still cannot solve the problem of the resistance of the end plug, and the spheroidal design of the front end of the end plug also will increase the amount of the residual fluid.

Though the US Patent with a grant announcement number of US6379336 B1 improves the structure of the self-destruction safety syringe, the structure of the syringe in this patent is quite complex and not easy to be assembled, moreover, the cost is high and the volume of the fluid residual is high, which is unacceptable when the injection fluid is expensive.

WO 2006/119570 A1 relates to a retractable syringe and a plunger, wherein the syringe has a barrel, a retractable needle mount to which is mounted or mountable a needle and wherein the plunger comprises an initially compressed spring, a means for engaging the retractable needle mount, an integrally formed plunger seal and a removable controlling means for facilitating control of the rate of retraction of needle mount when engaged with plunger.

Although a lot of self-destruction safety syringes have been developed, these syringes still have various structure defects, especially when the connection structures form an interference fit, the problem that the residual fluid is sealed has not been solved yet all the time. The present invention makes further improvement to the structure of the self-destruction safety syringe on the basis of the existing technology to solve the above technical problem.

### Summary of the Invention

The objective of the present invention is to provide a disposable self-destruction safety syringe without fluid residua, in which, the needle mount together with the needle can safely and automatically retract into the syringe tube and it is realized that no fluid residua left in the syringe tube.

The present invention is accomplished by the following technical solutions: a disposable self-destruction safety syringe without fluid residua, comprising a syringe tube, a push rod, a needle mount and a needle, the needle being mounted on the needle mount, the needle mount and the push rod being mounted in the syringe tube, a plunger slidably fitting with the inside wall of the syringe tube being further provided on the front end of the push rod, wherein an automatic retraction means is mounted in the front end of the chamber of the push rod; the automatic retraction means comprises a retraction rod, a compression spring socket-jointed on the retraction rod, an elastic rubber washer mounted on the terminal end of the retraction rod and a position limiting ring provided on the inside wall of the push rod. The elastic rubber washer and the position limiting ring form an interference fit with each other so as to fix the automatic retraction means in the front end of the chamber of the push rod. The retraction rod of the automatic retraction means and the needle mount are each provided with a snap-latch member, respectively, the two snap-latch members form an interference fit with each other for connecting the needle mount with the automatic retraction means; each of the snap-latch member is provided with fluid pass channels such that the fluid remaining inside the syringe tube flows, through the fluid pass channels when the snap-latch members form interference fit and engage with each other, into the chamber of the needle mount and the needle.

A fluid-stop ring is further provided between the automatic retraction means and the front end of the chamber of the push rod for sealing the gap between the automatic retraction means and the chamber of the push rod to prevent fluid leakage.

As an optional solution, the needle mount is provided with a first step, a second step and a third step; the first step and the tube hole on the top end of the syringe tube fit with each other to prevent incline of the needle during injection; a hook step is provided between the second step and the third step to prevent back-off of the needle mount when a force is exerted on the needle mount during injection; and the snap-latch member is provided on the third step.

As an optional solution, the inside wall of the needle mount is further provided with a ledge as a position limit for mounting the needle.

The syringe tube is provided inside with a fluid-stop ring socket-jointed on the needle mount for fixing the needle mount and sealing the gap between the needle mount and the inside wall of the syringe tube to prevent fluid leakage. The front surface of the fluid-stop ring forms a chamber with the inside wall of the syringe tube as space for the fluid-stop ring to slide forward and disengage from the needle mount.

As an optional solution, the circumference of the top end of the push rod is provided with annular top block or blocks for pushing the fluid-stop ring in the syringe tube to slide forward to disengage from the needle mount; and the annular top block is provided with fluid pass channels such that the fluid remaining inside the syringe tube flows, through the fluid pass channels when the annular top block is tangent to and engaged with the fluid-stop ring, into the chamber of the needle mount and the needle.

The inside wall of the syringe tube is further provided with a convex retaining ring at the rear end of the fluid-stop ring for preventing back-off of the needle mount when a force is exerted on the needle mount during injection. This convex retaining ring forms a double-layered resistance together with the hook-step provided between the second and third steps of the needle mount for effectively preventing back-off of the needle mount when a force is exerted on the needle mount during injection.

The plunger, the elastic rubber washer and the fluid-stop ring are made by the synthetic rubber materials.

The self-destruction safety syringe according to the present invention further comprises a needle socket-jointed on the front end of the syringe tube and a top plug provided at the terminal end of the push rod.

The self-destruction safety syringe according to the present invention makes improvements on the structure. By the action of the interference fit between the elastic rubber washer and the position limiting ring, the automatic retraction means is fixed on the front end of the chamber of the push rod, and when the injection is finished, the elastic apron is disengaged from the position limiting ring by a counterforce, the retraction rod together with the needle mount and the needle is pushed and retract into the syringe tube under the action of the elasticity of the spring so as to realize disposable automatic and safe self-destruction. Moreover, by providing the fluid pass channels on the snap-latch members of the automatic retraction means and the needle mount, the fluid remaining inside the syringe tube can continue to flow into the chamber of the needle mount and the needle, and therefore be injected into the human body when the automatic retraction means and the needle mount form an interference fit and engage with each other, therefore, no fluid residua inside the syringe tube is realized. Further, the double-layered resistance structure is formed by providing the hook-step on the needle mount and providing the convex retaining ring on the inside wall of the syringe tube, which is capable of effectively preventing back-off of the needle mount when a force is exerted on the needle mount during injection. Further, by providing the first step slidably fitting with the tube hole on the top end of the syringe tube on the needle mount, the incline of the needle in injection can be prevented.

### Description of the Accompanying Drawings

Figure 1 is a structural scheme of the syringe of embodiment 1 of the present invention;
Figure 2 is a structural scheme of the syringe of embodiment 1 of the present invention when the needle mount and the needle have retracted into the syringe tube;
Figure 3 is a structural scheme of the needle mount of embodiment 1 of the present invention;
Figure 3a is a bottom view of Figure 3;
Figure 4 is a structural scheme of the retraction rod of embodiment 1 of the present invention;
Figure 4a is a top view of Figure 4;
Figure 5 is a structural scheme of the syringe of embodiment 2 of the present invention;
Figure 6 is a structural scheme of the syringe of embodiment 2 of the present invention when the needle mount and the needle have retracted into the syringe tube;
Figure 7 is a structural scheme of the needle mount of embodiment 2 of the present invention;
Figure 7a is a bottom view of Figure 7;
Figure 8 is a structural scheme of the retraction rod of embodiment 2;
Figure 8a is a top view of Figure 8;
Figure 9 is a structural scheme of the push rod of embodiment 2 of the present invention;
Figure 9a is a top view of Figure 9;
Figure 10 is a structural scheme of the syringe of embodiment 3 of the present invention;
Figure 11 is a structural scheme of the syringe of embodiment 3 of the present invention when the needle mount and the needle have retracted into the syringe tube;
Figure 12 is a structural scheme of the needle mount of embodiment 3 of the present invention;
Figure 13 is a structural scheme of the retraction rod of embodiment 3 of the present invention;
Figure 14 is a structural scheme of the syringe of embodiment 4 of the present invention;
Figure 15 is a structural scheme of the syringe of embodiment 4 of the present invention when the needle mount and the needle have retracted into the syringe tube;
Figure 16 is a structural scheme of the needle mount of embodiment 4 of the present invention;
Figure 16a is a bottom view of Figure 16;
Figure 17 is a structural scheme of the retraction rod of embodiment 4 of the present invention;
Figure 17a is a top view of Figure 17; and
Figure 18 is a structural scheme of the push rod of embodiment 4 of the present invention.

### Detailed Description of Embodiments

### Embodiment 1:

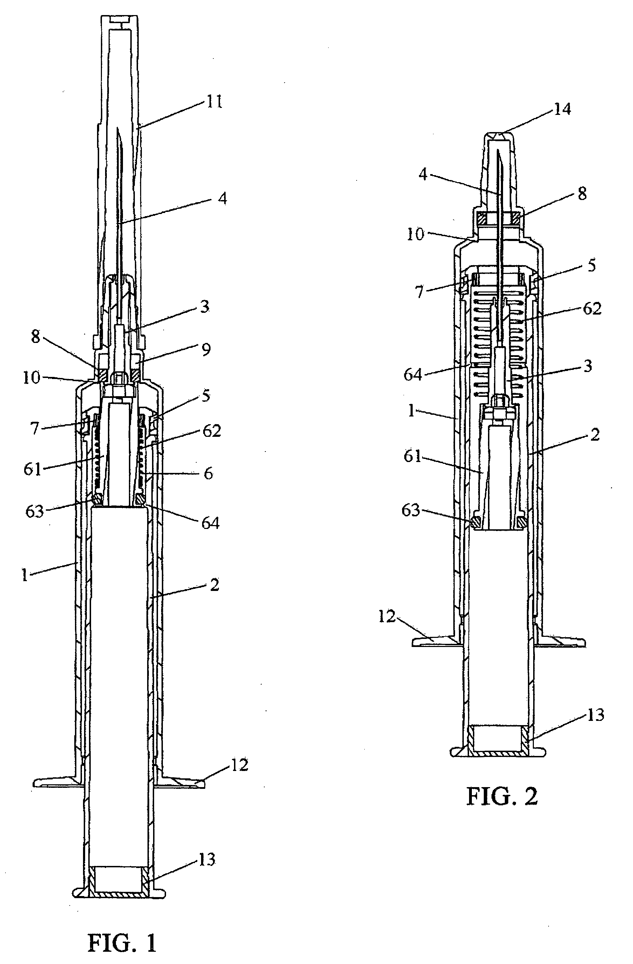

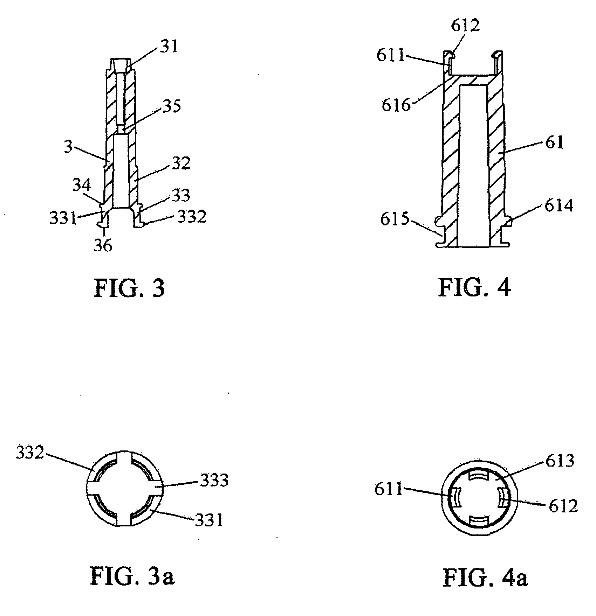

As shown in Figure 1 to Figure 4, the self-destruction safety syringe according to the present invention comprises a syringe tube 1, a push rod 2, a needle mount 3, a needle 4, a needle cannula 11, a handle 12 and a top plug 13, the needle 4 being mounted on the needle mount 3, the needle mount 3 and the push rod 2 being mounted in the syringe tube 1, a plunger 5 slidably fitting with the inside wall of the syringe tube 1 being further provided on the front end of the push rod 2.

An automatic retraction means 6 is mounted in the front end of the chamber of the push rod 2. The automatic retraction means 6 comprises a retraction rod 61, a compression spring 62, an elastic rubber washer 63 and a position limiting ring 64. A fluid-stop ring 7 is further provided between the front end of the automatic retraction means 6 and the front end of the chamber of the push rod 2. The front end of the automatic retraction means 6 is tightly press-engaged on the fluid-stop ring 7 so as to seal the gap between the automatic retraction means 6 and the chamber of the push rod 2 to prevent fluid leakage. The circumference of the top end of the retraction rod 61 is provided with four annular sector-shaped top blocks 611 arranged with equidistant intervals. The top end of each annular sector-shaped top block 611 is further provided with an inner shackle 612. Fluid pass channels 613 are formed among the annular sector-shaped top blocks 611. The terminal end of the retraction rod 61 is provided with a ledge 614, a groove 615 is formed in the middle of the ledge 614, and the elastic rubber washer 63 is placed in the groove 615. The compression spring 62 is socket-jointed on the retraction rod 61 with its front end engaged with the terminal end of the fluid-stop ring 7 and its rear end engaged with the front surface of the ledge 614 of the retraction rod 61. The position limiting ring 64 is provided on the inside wall of the push rod 2 to form an interference fit with the elastic rubber washer 63 so as to fix the automatic retraction means 6 in the front end of the chamber of the push rod 2.

The needle mount 3 is provided with a first step 31, a second step 32 and a third step 33. The first step 31 slidably fits with the tube hole 14 in the center of the top end of the syringe tube 1 for preventing incline of the needle during injection. A hook step 34 is provided between the second step 32 and the third step 33 to prevent back-off of the needle mount when a force is exerted on the needle mount during injection. The third step 33 is formed by four annular sector-shaped top blocks 331 arranged with equidistant intervals. The top end of each annular sector-shaped top block 331 is further provided with an outer shackle 332. Fluid pass channels 333 are formed among the annular sector-shaped top blocks 331. The annular sector-shaped top blocks 331 of the needle mount 3 and the annular sector-shaped top blocks 611 of the automatic retraction means 6 are arranged corresponding to each other, and the outer shackles 332 on the annular sector-shaped top blocks 331 and the inner shackles 612 on the annular sector-shaped top blocks 611 form an interference fit with each other. When the inner shackles 612 and the outer shackles 332 are snap-latched to each other, the needle mount 3 and the automatic retraction means 6 can be connected to each other. Moreover, the inside wall of the needle mount 3 is further provided with a ledge 35 as a position limit for mounting the needle 4.

The syringe tube 1 is equipped with a fluid-stop ring 8 socket-jointed on the third step 33 of the needle mount 3 for fixing the needle mount 3 and sealing the gap between the needle mount 3 and the inside wall of the syringe tube 1 to prevent fluid leakage. The fluid-stop ring 8 has the same height as the third step 33 and has its front end tangent to the hook step 34 provided between the second step 32 and the third step 33 of the needle mount 3 so as to enhance its fixation effect to the needle mount 3. The front surface of the fluid-stop ring 8 further forms a chamber 9 with the inside wall of the syringe tube 1 as space for the fluid-stop ring 8 to slide forward and disengage from the needle mount 3. In addition, the inside wall of the syringe tube 1 is further provided with a convex retaining ring 10 at the rear end of the fluid-stop ring 8 for fixing the location of the fluid-stop ring 8 and preventing back-off of the needle mount during injection when a force is exerted on the needle mount. The convex retaining ring 10 and the hook step 34 provided between the second step 32 and the third step 33 of the needle mount 3 form a double-layered resistance for effectively preventing back-off of the needle mount when a force is exerted on the needle mount during injection.

During injection, as the push rod 2 is pushed forward, the fluid in the syringe tube 1 flows into the needle 4 through the chamber of the needle mount 3 and is injected into the human body or animal body through the needle 4. When the push rod 2 is pushed to the front end of the syringe tube 1 and the annular sector-shaped top blocks 611 on the automatic retraction means 6 are tangent to the annular sector-shaped top blocks 331 on the needle mount 3, the inner shackles 612 on the annular sector-shaped top blocks 611 and the outer shackles 332 on the annular sector-shaped top blocks 331 are snap-latched to each other under the action of thrust so as to connect the needle mount 3 with the automatic retraction means 6. Meanwhile, the annular sector-shaped top blocks 611 are tangent to the rear surface of the fluid-stop ring 8, so that the fluid-stop ring 8 is then pushed forward to slide forward and disengage from the third step 33 of the needle mount 3 and to enter into the chamber 9. In the present embodiment, since the snap-latch member is formed by four annular sector-shaped top blocks arranged with equidistant intervals having shackles, the resistance during the snap-latch is then quartered, and there will be almost no resistance sensed when the push rod is pushed for snap-latch. Moreover, since the fluid pass channels are formed among the annular sector-shaped top blocks, the fluid remaining in the syringe tube can still flow, through the fluid pass channels when the automatic retraction means is tangent to and engaged with the needle mount, into the chamber of the needle mount 3 and the needle 4, therefore, there is no fluid residua in the syringe tube. After the inner shackles 612 and the outer shackles 332 are snap-latched to each other, continue to push the push rod 2 so that the front end top surface 616 of the retraction rod 61 presses on the terminal end top surface 36 of the needle mount 3, at this moment, the terminal end top surface 36 of the needle mount 3 will exert a counterforce to the front end top surface 616 of the retraction rod 61 forcing the elastic rubber washer 63 of the automatic retraction means 6 to disengage from the position limiting ring 64, and then, the compression spring 62 releases the elastic force to push the retraction rod 61 to retract together with the needle mount 3 and the needle 4 into the syringe tube 1.

### Embodiment 2:

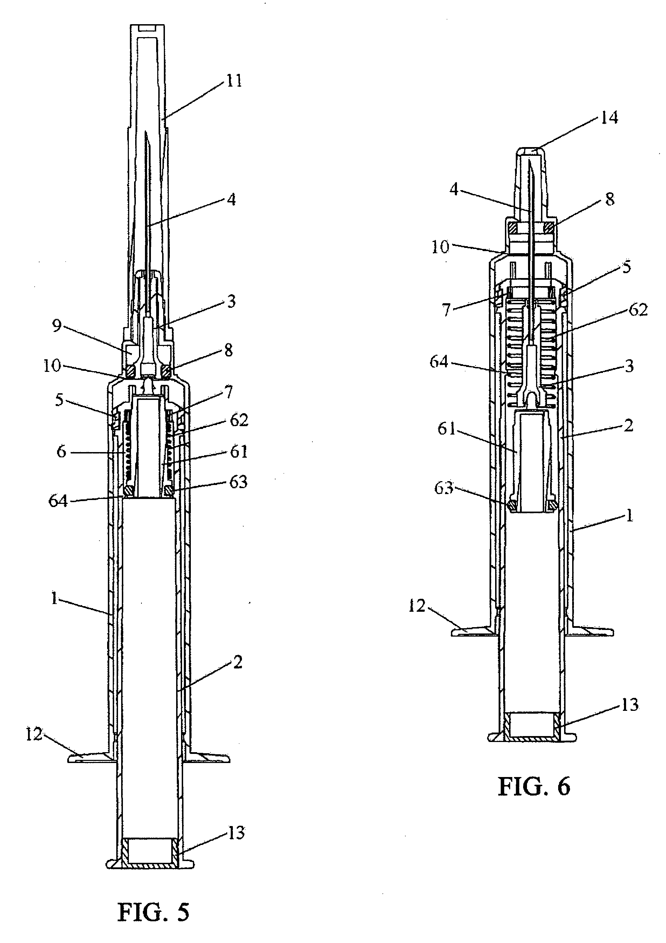

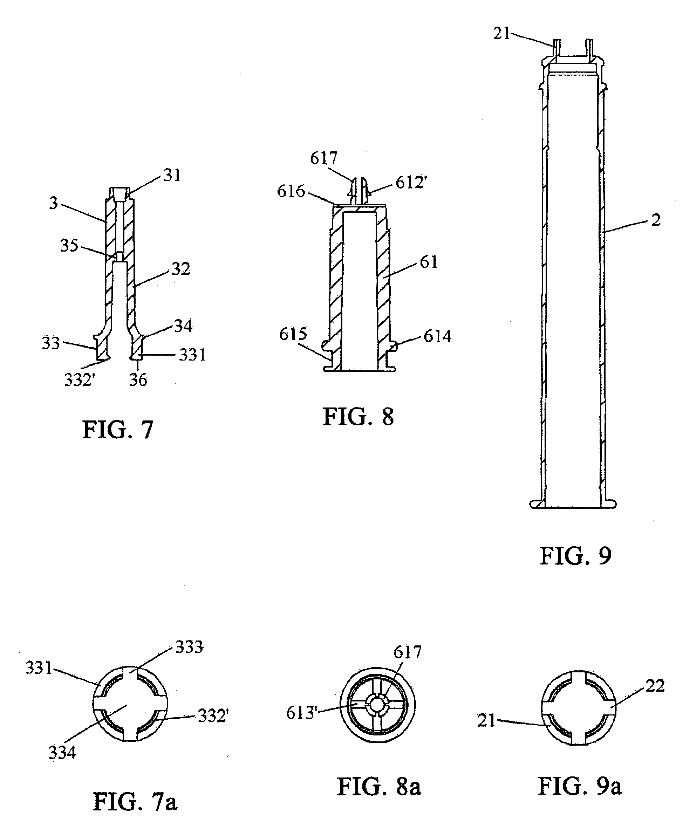

As shown in Figure 5 to Figure 9, the self-destruction safety syringe according to the present invention comprises a syringe tube 1, a push rod 2, a needle mount 3, a needle 4, a needle cannula 11, a handle 12 and a top plug 13, the needle 4 being mounted on the needle mount 3, the needle mount 3 and the push rod 2 being mounted in the syringe tube 1, a plunger 5 slidably fitting with the inside wall of the syringe tube 1 being further provided on the front end of the push rod 2.

An automatic retraction means 6 is mounted in the front end of the chamber of the push rod 2. The automatic retraction means 6 comprises a retraction rod 61, a compression spring 62, an elastic rubber washer 63 and a position limiting ring 64. A fluid-stop ring 7 is further provided between the front end of the automatic retraction means 6 and the front end of the chamber of the push rod 2. The front end of the automatic retraction means 6 is tightly press-engaged on the fluid-stop ring 7 so as to seal the gap between the automatic retraction means 6 and the chamber of the push rod 2 to prevent fluid leakage. The top center of the retraction rod 61 is provided with four spheroidal top blocks 617 arranged with equidistant intervals. The center of the throat of each spheroidal top block 617 is further provided with an outer shackle 612'. Fluid pass channels 613' are formed among the spheroidal top blocks 617. The terminal end of the retraction rod 61 is provided with a ledge 614, a groove 615 is formed in the middle of the ledge 614, and the elastic rubber washer 63 is placed in the groove 615. The compression spring 62 is socket-jointed on the retraction rod 61 with its front end engaged with the terminal end of the fluid-stop ring 7 and its rear end engaged with the front surface of the ledge 614 of the retraction rod 61. The position limiting ring 64 is provided on the inside wall of the push rod 2 to form an interference fit with the elastic rubber washer 63 so as to fix the automatic retraction means 6 in the front end of the chamber of the push rod 2. In addition, the circumference of the top end of the push rod 2 is further provided with four annular sector-shaped top blocks 21 arranged with equidistant intervals, and the fluid pass channels 22 are formed among the annular sector-shaped top blocks 21.

The needle mount 3 is provided with a first step 31, a second step 32 and a third step 33. The first step 31 slidably fits with the tube hole 14 in the center of the top end of the syringe tube 1 for preventing incline of the needle during injection. A hook step 34 is provided between the second step 32 and the third step 33 to prevent back-off of the needle mount when a force is exerted on the needle mount during injection. The third step 33 is formed by four annular sector-shaped top blocks 331 arranged with equidistant intervals. The top ends of the annular sector-shaped top blocks 331 are further provided with inner shackles 332'. Fluid pass channels 333 are formed among the annular sector-shaped top blocks 331, and the four inner shackles 332' form a clamping chamber 334. The annular sector-shaped top blocks 331 of the needle mount 3 and the spheroidal top blocks 617 of the automatic retraction means 6 are arranged corresponding to each other, and the inner shackles 332' on the annular sector-shaped top blocks 331 and the outer shackles 612' on the spheroidal top blocks 617 form an interference fit with each other. When the outer shackles 612' and the inner shackles 332' are snap-latched to each other, the needle mount 3 can be connected with the automatic retraction means 6. Moreover, the inside wall of the needle mount 3 is further provided with a ledge 35 as a position limit for mounting the needle 4.

The syringe tube 1 is equipped with a fluid-stop ring 8 socket-jointed on the third step 33 of the needle mount 3 for fixing the needle mount 3 and sealing the gap between the needle mount 3 and the inside wall of the syringe tube 1 to prevent fluid leakage. The fluid-stop ring 8 has the same height as the third step 33 and has its front end tangent to the hook step 34 provided between the second step 32 and the third step 33 of the needle mount 3 so as to enhance its fixation effect to the needle mount 3. The front surface of the fluid-stop ring 8 further forms a chamber 9 with the inside wall of the syringe tube 1 as the space for the fluid-stop ring 8 to slide forward and disengage from the needle mount 3. In addition, the inside wall of the syringe tube 1 is further provided with a convex retaining ring 10 at the rear end of the fluid-stop ring 8 for fixing the location of the fluid-stop ring 8 and preventing back-off of the needle mount when a force is exerted on the needle mount during injection. The convex retaining ring 10 and the hook step 34 provided between the second step 32 and the third step 33 of the needle mount 3 form a double-layered resistance for effectively preventing back-off of the needle mount when a force is exerted on the needle mount during injection.

During injection, as the push rod 2 is pushed forward, the fluid in the syringe tube 1 flows into the needle 4 through the chamber of the needle mount 3 and is injected into the human body or animal body through the needle 4. When the push rod 2 is pushed to the front end of the syringe tube 1, the spheroidal top blocks 617 on the automatic retraction means 6 enter into the clamping chamber 334 in the needle mount 3, and the outer shackles 612' on the spheroidal top blocks 617 and the inner shackles 332' on the annular sector-shaped top blocks 331 are snap-latched to each other under the action of thrust so as to connect the needle mount 3 with the automatic retraction means 6. Meanwhile, the annular sector-shaped top blocks 21 on the push rod 2 are tangent to the rear surface of the fluid-stop ring 8, so that the fluid-stop ring 8 is then pushed forward to slide forward and disengage from the third step 33 of the needle mount 3 and to enter into the chamber 9. In the present embodiment, since the snap-latch member is formed by four annular sector-shaped top blocks arranged with equidistant interval having shackles and spheroidal top blocks, the resistance during the snap-latch is then quartered, and there will be almost no resistance sensed when the push rod is pushed for snap-latch. Moreover, since the fluid pass channels are formed between the annular sector-shaped top blocks on the push rod and the needle mount and the spheroidal top blocks on the automatic retraction means, the fluid remaining in the syringe tube can still flow, through the fluid pass channels when the automatic retraction means is engaged with the needle mount, into the chamber of the needle mount 3 and the needle 4, therefore, there is no fluid residua in the syringe tube. After the outer shackles 612' and the inner shackles 332' are snap-latched to each other, continue to push the push rod 2 so that the front end top surface 616 of the retraction rod 61 press on the terminal end top surface 36 of the needle mount 3, at this moment, the terminal end top surface 36 of the needle mount 3 will exert a counterforce to the front end top surface 616 of the retraction rod 61 forcing the elastic rubber washer 63 of the automatic retraction means 6 to disengage from the position limiting ring 64, and then, the compression spring 62 releases the elastic force to push the retraction rod 61 to retract together with the needle mount 3 and the needle 4 into the syringe tube 1.

### Embodiment 3:

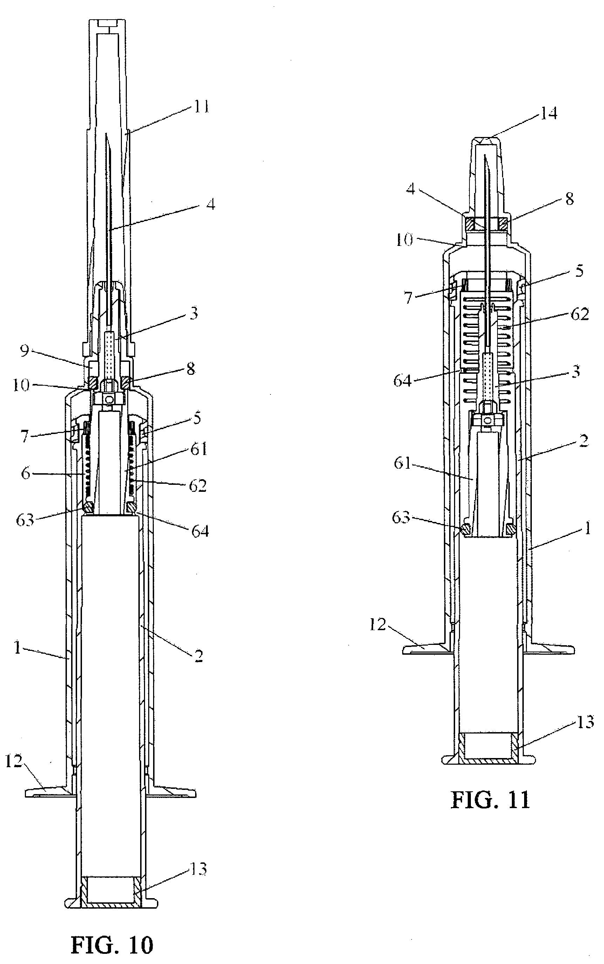

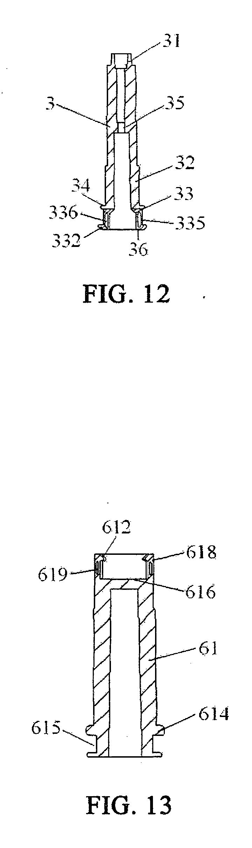

As shown in Figure 10 to Figure 13, the self-destruction safety syringe according to the present invention comprises a syringe tube 1, a push rod 2, a needle mount 3, a needle 4, a needle cannula 11, a handle 12 and a top plug 13, the needle 4 being mounted on the needle mount 3, the needle mount 3 and the push rod 2 being mounted in the syringe tube 1, a plunger 5 slidably fitting with the inside wall of the syringe tube 1 being further provided on the front end of the push rod 2.

An automatic retraction means 6 is mounted in the front end of the chamber of the push rod 2. The automatic retraction means6 comprises a retraction rod 61, a compression spring 62, an elastic rubber washer 63 and a position limiting ring 64. A fluid-stop ring 7 is further provided between the front end of the automatic retraction means6 and the front end of the chamber of the push rod 2. The front end of the automatic retraction means 6 is tightly press-engaged on the fluid-stop ring 7 so as to seal the gap between the automatic retraction means 6 and the chamber of the push rod 2 to prevent fluid leakage. The circumference of the top end of the retraction rod 61 is provided with an annular top block 618, the top end of the annular top block 618 is provided with an inner shackle 612, and a fluid pass hole 619 is further formed on the wall of the annular top block 618. The terminal end of the retraction rod 61 is provided with a ledge 614, a groove 615 is formed in the middle of the ledge 614, and the elastic rubber washer 63 is placed in the groove 615. The compression spring 62 is socket-jointed on the retraction rod 61 and with its front end engaged with the terminal end of the fluid-stop ring 7 and its rear end engaged with the front surface of the ledge 614 of the retraction rod 61. The position limiting ring 64 is provided on the inside wall of the push rod 2 to form an interference fit with the elastic rubber washer 63 so as to fix the automatic retraction means6 in the front end of the chamber of the push rod 2.

The needle mount 3 is provided with a first step 31, a second step 32 and a third step 33. The first step 31 slidably fits with the tube hole 14 in the center of the top end of the syringe tube 1 for preventing incline of the needle during injection. A hook step 34 is provided between the second step 32 and the third step 33 to prevent back-off of the needle mount when a force is exerted on the needle mount during injection. The third step 33 is formed by an annular top block 335, the top end of the annular top block 335 is provided with an outer shackle 332, and a fluid pass hole 336 is further formed on the wall of the annular top block 335. The outer shackle 332 on the annular top block 335 and the inner shackle 612 on the annular top block 618 form an interference fit with each other. When the inner shackle 612 and the outer shackle 332 are snap-latched to each other, the needle mount 3 can be connected with the automatic retraction means 6. Moreover, the inside wall of the needle mount 3 is further provided with a ledge 35 as a position limit for mounting the needle 4.

The syringe tube 1 is equipped with a fluid-stop ring 8 socket-jointed on the third step 33 of the needle mount 3 for fixing the needle mount 3 and sealing the gap between the needle mount 3 and the inside wall of the syringe tube 1 to prevent fluid leakage. The fluid-stop ring 8 has the same height as the third step 33 and has its front end tangent to the hook step 34 provided between the second step 32 and the third step 33 of the needle mount 3 so as to enhance its fixation effect to the needle mount 3. The front surface of the fluid-stop ring 8 further forms a chamber 9 with the inside wall of the syringe tube 1 as space for the fluid-stop ring 8 to slide forward and disengage from the needle mount 3. In addition, the inside wall of the syringe tube 1 is further provided with a convex retaining ring 10 at the rear end of the fluid-stop ring 8 for fixing the location of the fluid-stop ring 8 and preventing back-off of the needle mount when a force is exerted on the needle mount during injection. The convex retaining ring 10 and the hook step 34 provided between the second step 32 and the third step 33 of the needle mount 3 form a double-layered resistance for effectively preventing back-off of the needle mount when a force is exerted on the needle mount during injection.

During injection, as the push rod 2 is pushed forward, the fluid in the syringe tube 1 flows into the needle 4 through the chamber of the needle mount 3 and is injected into the human body or animal body through the needle 4. When the push rod 2 is pushed to the front end of the syringe tube 1 and the annular top block 618 on the automatic retraction means 6 is tangent to the annular top block 335 on the needle mount 3, the inner shackle 612 on the annular top block 618 and the outer shackle 332 on the annular top block 335 are snap-latched to each other under the action of thrust so as to connect the needle mount 3 with the automatic retraction means 6. Meanwhile, the annular top block 618 is tangent to the rear surface of the fluid-stop ring 8, so that the fluid-stop ring 8 is then pushed forward to slide forward and disengage from the third step 33 of the needle mount 3 and to enter into the chamber 9. In the present embodiment, since the fluid pass hole is formed on the wall of the annular top block, the fluid remaining in the syringe tube can still flow, through the fluid pass hole when the automatic retraction means is tangent to and engaged with the needle mount, into the chamber of the needle mount 3 and the needle 4, therefore, there is no fluid residua in the syringe tube. After the inner shackle 612 and the outer shackle 332 are snap-latched to each other, continue to push the push rod 2 so that the front end top surface 616 of the retraction rod 61 presses on the terminal end top surface 36 of the needle mount 3, at this moment, the terminal end top surface 36 of the needle mount 3 will exert a counterforce to the front end top surface 616 of the retraction rod 61 forcing the elastic rubber washer 63 of the automatic retraction means6 to disengage from the position limiting ring 64, and then, the compression spring 62 releases the elastic force to push the retraction rod 61 to retract together with the needle mount 3 and the needle 4 into the syringe tube 1.

### Embodiment 4:

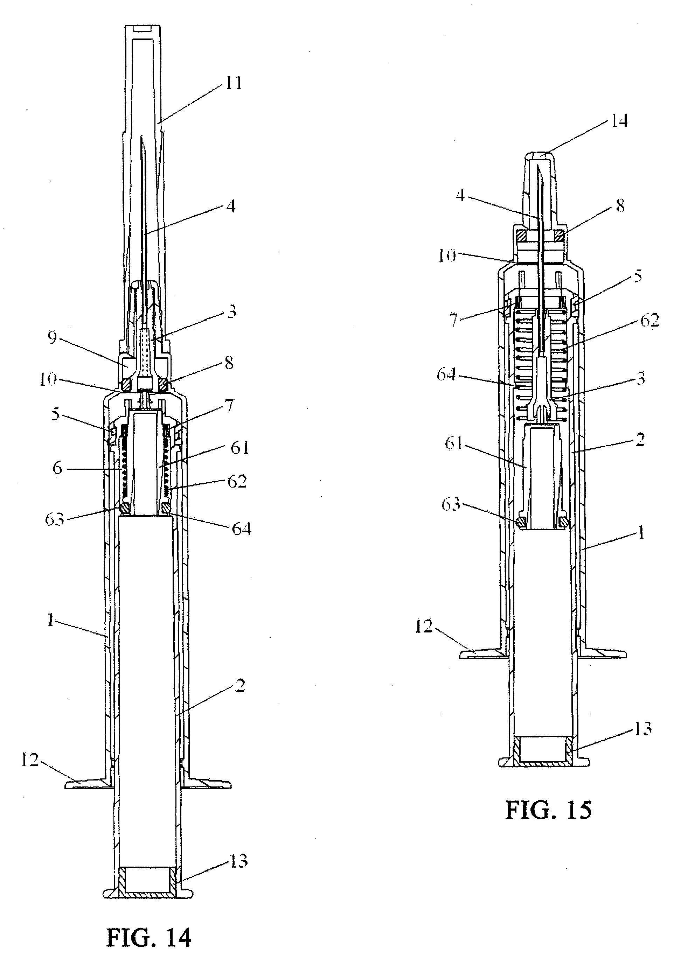

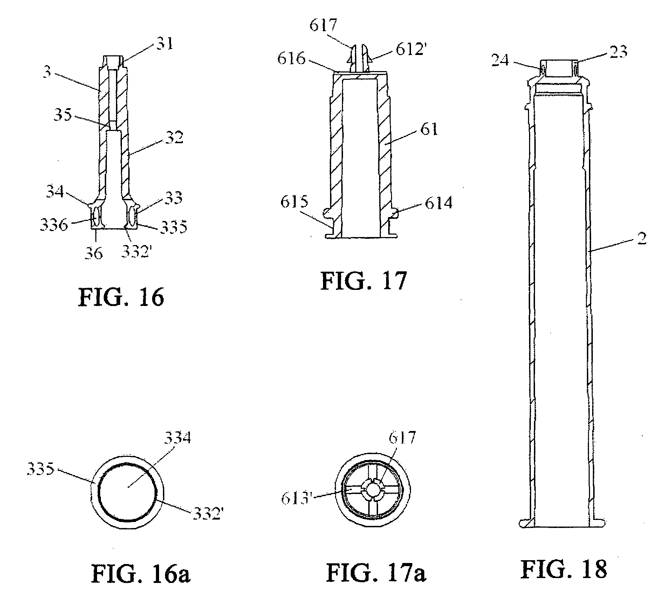

As shown in Figure 14 to Figure 18, the self-destruction safety syringe according to the present invention comprises a syringe tube 1, a push rod 2, a needle mount 3, a needle 4, a needle cannula 11, a handle 12 and a top plug 13, the needle 4 being mounted on the needle mount 3, the needle mount 3 and the push rod 2 being mounted in the syringe tube 1, a plunger 5 slidably fitting with the inside wall of the syringe tube 1 being further provided on the front end of the push rod 2.

An automatic retraction means 6 is mounted in the front end of the chamber of the push rod 2. The automatic retraction means 6 comprises a retraction rod 61, a compression spring 62, an elastic rubber washer 63 and a position limiting ring 64. A fluid-stop ring 7 is further provided between the front end of the automatic retraction means 6 and the front end of the chamber of the push rod 2. The front end of the automatic retraction means 6 is tightly press-engaged on the fluid-stop ring 7 so as to seal the gap between the automatic retraction means 6 and the chamber of the push rod 2 to prevent fluid leakage. The top center of the retraction rod 61 is provided with four spheroidal top blocks 617 arranged with equidistant intervals. The center of the throat of each spheroidal top block 617 is further provided with an outer shackle 612'. Fluid pass channels 613' are formed among the spheroidal top blocks 617. The terminal end of the retraction rod 61 is provided with a ledge 614, a groove 615 is formed in the middle of the ledge 614, and the elastic rubber washer 63 is placed in the groove 615. The compression spring 62 is socket-jointed on the retraction rod 61 with its front end engaged with the terminal end of the fluid-stop ring 7 and its rear end engaged with the front surface of the ledge 614 of the retraction rod 61. The position limiting ring 64 is provided on the inside wall of the push rod 2 to form an interfere fit with the elastic rubber washer 63 so as to fix the automatic retraction means6 in the front end of the chamber of the push rod 2. In addition, the circumference of the top end of the push rod 2 is provided with an annular top block 23, and a fluid pass hole 24 is further formed on the wall of the annular top block 23.

The needle mount 3 is provided with a first step 31, a second step 32 and a third step 33. The first step 31 slidably fits with the tube hole 14 in the center of the top end of the syringe tube 1 for preventing incline of the needle during injection. A hook step 34 is provided between the second step 32 and the third step 33 to prevent back-off of the needle mount when a force is exerted on the needle mount during injection. The third step 33 is formed by an annular top block 335, the top end of the annular top block 335 is provided with an inner shackle 332', a fluid pass hole 336 is further formed on the wall of the annular top block 335, and the inner shackle 32 forms a clamping chamber 334. The fluid pass hole 336 on the annular top block 335 and the fluid pass channels 613' among the spheroidal top blocks 617 are arranged corresponding to each other, and the inner shackle 332' on the annular top block 335 and the outer shackles 612' on the spheroidal top blocks 617 form an interference fit with each other. When the outer shackles 612' and the inner shackle 332' are snap-latched with each other, the needle mount 3 can be connected with the automatic retraction means 6. Moreover, the inside wall of the needle mount 3 is further provided with a ledge 35 as a position limit for mounting the needle 4.

The syringe tube 1 is equipped with a fluid-stop ring 8 socket-jointed on the third step 33 of the needle mount 3 for fixing the needle mount 3 and sealing the gap between the needle mount 3 and the inside wall of the syringe tube 1 to prevent fluid leakage. The fluid-stop ring 8 has the same height as the third step 33 and has its front end tangent to the hook step 34 provided between the second step 32 and the third step 33 of the needle mount 3 so as to enhance its fixation effect to the needle mount 3. The front surface of the fluid-stop ring 8 further forms a chamber 9 with the inside wall of the syringe tube 1 as space for the fluid-stop ring 8 to slide forward and disengage from the needle mount 3. In addition, the inside wall of the syringe tube 1 is further provided with a convex retaining ring 10 at the rear end of the fluid-stop ring 8 for fixing the location of the fluid-stop ring 8 and preventing back-off of the needle mount when a force is exerted on the needle mount during injection. The convex retaining ring 10 and the hook step 34 provided between the second step 32 and the third step 33 of the needle mount 3 form a double-layered resistance for effectively preventing back-off of the needle mount when a force is exerted on the needle mount during injection.

During injection, as the push rod 2 is pushed forward, the fluid in the syringe tube 1 flows into the needle 4 through the chamber of the needle mount 3 and is injected into the human body or animal body through the needle 4. When the push rod 2 is pushed to the front end of the syringe tube 1, the spheroidal top blocks 617 on the automatic retraction means 6 enter into the clamping chamber 334 in the needle mount 3, and the outer shackles 612' on the spheroidal top blocks 617 and the inner shackle 332' on the annular top block 335 are snap-latched to each other under the action of thrust so as to connect the needle mount 3 with the automatic retraction means 6. Meanwhile, the annular top block 23 on the push rod 2 is tangent to the rear surface of the fluid-stop ring 8, so that the fluid-stop ring 8 is then pushed forward to slide forward and disengage from the third step 33 of the needle mount 3 and to enter into the chamber 9. In the present embodiment, since the fluid pass holes are formed on the wall of the annular top block of the push rod and on the wall of the annular top block on the needle mount, and the fluid pass channels are formed among the spheroidal top blocks on the automatic retraction means, the fluid remaining in the syringe tube can still flow, through the fluid pass holes and the fluid pass channels when the automatic retraction means is engaged with the needle mount, into the chamber of the needle mount 3 and the needle 4, therefore, there is no fluid residua in the syringe tube. After the outer shackles 612' and the inner shackle 332' are snap-latched to each other, continue to push the push rod 2 so that the front end top surface 616 of the retraction rod 61 presses on the terminal end top surface 36 of the needle mount 3, at this moment, the terminal end top surface 36 of the needle mount 3 will exert a counterforce to the front end top surface 616 of the retraction rod 61 forcing the elastic rubber washer 63 of the automatic retraction means 6 to disengage from the position limiting ring 64, and then, the compressive spring 62 releases the elastic force to push the retraction rod 61 to retract together with the needle mount 3 and the needle 4 into the syringe tube 1.

The present invention includes but not limits to the contents in the above embodiments. All the other product structures having substantively the same content as that in the claims of the present invention should fall into the scope of protection of the present invention.

## Claims

1. A disposable self-destruction safety syringe without fluid residua, comprising a syringe tube (1), a push rod (2), a needle mount (3) and a needle (4), the needle (4) being mounted on the needle mount (3), the needle mount (3) and the push rod (2) being mounted in the syringe tube (1), a plunger (5) slidably fitting with an inside wall of the syringe tube (1) being further provided on a front end of the push rod (2), an automatic retraction means (6) being mounted in a front end of a chamber of the push rod (2), the automatic retraction means (6) comprising a retraction rod (61) and a compression spring (62) socket-jointed on the retraction rod (61), the retraction rod (61) of the automatic retraction means (6) and the needle mount (3) each being provided with a snap-latch member (612; 332), respectively, the two snap-latch members (612; 332) form an interference fit with each other for connecting the needle mount (3) with the automatic retraction means (6); **characterized in that**: an elastic rubber washer (63) is mounted on a terminal end of the retraction rod (61) and a position limiting ring (64) is provided on an inside wall of the push rod (2), the elastic rubber washer (63) and the position limiting ring (64) forming an interference fit with each other to fix the automatic retraction means (6) in the front end of the chamber of the push rod (2); each of the snap-latch members (612; 332) is provided with fluid pass channels (613; 333) such that fluid remaining inside the syringe tube (1) can still flow through the fluid pass channels (613; 333) and into a chamber of the needle mount (3) and the needle (4) when the snap-latch members (612; 332) form interference fit and engage with each other.

2. The self-destruction safety syringe according to Claim 1, **characterized in that** a fluid-stop ring (8) is further provided between the automatic retraction means (6) and the front end of the chamber of the push rod (2) for sealing a gap between the automatic retraction means (6) and the chamber of the push rod (2) to prevent fluid leakage.

3. The self-destruction safety syringe according to Claim 1, **characterized in that**: the needle mount (3) is provided with a first step (31), a second step (32) and a third step (33); the first step (31) and a tube hole (14) on a top end of the syringe tube (1) fit with each other to prevent incline of the needle (4) during injection; a hook step (34) is provided between the second step (32) and the third step (33) to prevent back-off of the needle mount (3) when a force is exerted on the needle mount (3) during injection; and the snap-latch member (332) of the needle mount (3) is provided on the third step (33).

4. The self-destruction safety syringe according to Claim 3, **characterized in that** an inside wall of the needle mount (3) is further provided with a ledge (35) as a position limit for mounting the needle (4).

5. The self-destruction safety syringe according to Claim 1, **characterized in that** the syringe tube (1) is provided inside with a fluid-stop ring (8) socket-jointed on the needle mount (3) for fixing the needle mount (3) and sealing a gap between the needle mount (3) and the inside wall of the syringe tube (1) to prevent fluid leakage.

6. The self-destruction safety syringe according to Claim 5, **characterized in that** a front surface of the fluid-stop ring (8) forms a chamber with the inside wall of the syringe tube (1) as space for the fluid-stop ring (8) to slide forward and disengage from the needle mount (3).

7. The self-destruction safety syringe according to Claim 6, **characterized in that** the circumference of a top end of the push rod (2) is provided with an annular top block (21) for pushing the fluid-stop ring (8) forward to slide forward to disengage from the needle mount (3); and the annular top block is provided with fluid pass channels (22) such that the fluid remaining inside the syringe tube (1) flows, through the fluid pass channels (22) when the annular top block is tangent to and engaged with the fluid-stop ring (8), into a chamber of the needle mount (3) and the needle (4).

8. The self-destruction safety syringe according to Claim 5, **characterized in that** the inside wall of the syringe tube (1) is further provided with a convex retaining ring (10) at a rear end of the fluid-stop ring (8) for preventing back-off of the needle mount (3) when a force is exerted on the needle mount (3) during injection.

9. The self-destruction safety syringe according to any one of Claims 2 and 6 to 8, **characterized in that** the plunger (5), the elastic rubber washer (63) and the fluid-stop ring (8) are made by synthetic rubber materials.

10. The self-destruction safety syringe according to Claim 1, **characterized by** further comprising a needle cannula (11) socket-jointed on a front end of the syringe tube (1) and a top plug (13) provided at a terminal end of the push rod (2).

## Patentansprüche

1. Selbstzerstörende Wegwerf-Sicherheitsspritze ohne Flüssigkeitsrückstände, mit einer Spritzenröhre (1), einer Stößelstange (2), einer Nadelhalterung (3) und einer Nadel (4), wobei die Nadel (4) an der Nadelhalterung (3) angeordnet ist, die Nadelhalterung (3) und die Stößelstange (2) in der Spritzenröhre (1) angeordnet sind, ein Kolben (5), der sich mit einer Innenwand der Spritzenröhre (1) gleitfähig zusammenpasst, ferner an einem vorderen Ende der Stößelstange (2) vorgesehen ist, eine automatische Rückholeinrichtung (6) in einem vorderen Ende einer Kammer der Stößelstange (2) angeordnet ist, wobei die automatische Rückholeinrichtung (6) eine Rückholstange (61) und eine auf der Rückholstange (61) muffenartig angeordnete Druckfeder (62) aufweist, wobei die Rückholstange (61) der automatischen Rückholeinrichtung (6) und die Nadelhalterung (3) jeweils mit einem Einrastelement (612; 332) versehen sind, wobei die beiden Einrastelemente (612, 332) eine Presspassung miteinander bilden, um die Nadelhalterung (3) mit der automatischen Rückholeinrichtung (6) zu verbinden; **dadurch gekennzeichnet, dass** ein elastischer Gummidichtring (63) an einem Endabschnittsende der Rückholstange (61) angeordnet ist und ein Positionsbegrenzungsring (64) an einer Innenwand der Stößelstange (2) vorgesehen ist, wobei der elastische Gummidichtring (63) und der Positionsbegrenzungsring (64) eine Presspassung miteinander bilden, um die automatische Rückholeinrichtung (6) im vorderen Ende der Kammer der Stößelstange (2) festzuhalten, wobei jedes der Einrastelemente (612; 332) mit Flüssigkeitsdurchlasskanälen (613; 333) versehen ist, so dass im Innern der Spritzenröhre (1) verbleibende Flüssigkeit weiterhin durch die Flüssigkeitsdurchlasskanäle (613; 333) und in eine Kammer der Nadelhalterung (3) und in die Nadel (4) fließen kann, wenn die Einrastelemente (612; 332) eine Presspassung bilden und miteinander in Eingriff treten.

2. Selbstzerstörende Sicherheitsspritze nach Anspruch 1, **dadurch gekennzeichnet, dass** ferner zwischen der automatischen Rückholvorrichtung (6) und dem vorderen Ende der Kammer der Stößelstange (2) ein Flüssigkeitsstoppring (8) vorgesehen ist, um einen Spalt zwischen der automatischen Rückholeinrichtung (6) und der Kammer der Stößelstange (2) abzudichten, um ein Entweichen von Flüssigkeit zu verhindern.

3. Selbstzerstörende Sicherheitsspritze nach Anspruch 1, **dadurch gekennzeichnet, dass** die Nadelhalterung (3) mit einer ersten Stufe (31), einer zweiten Stufe (32) und einer dritten Stufe (33) versehen ist; die erste Stufe (31) und ein Röhrenloch (14) an einem oberen Ende der Spritzenröhre (1) sich zusammenfügen, um eine Neigung der Nadel (4) während der Injektion zu verhindern; eine Hakenstufe (34) zwischen der zweiten Stufe (32) und der dritten Stufe (33) vorgesehen ist, um ein Zurückweichen der Nadelhalterung (3) zu verhindern, wenn während der Injektion eine Kraft auf die Nadelhalterung (3) ausgeübt wird; und das Einrastelement (332) der Nadelhalterung (3) auf der dritten Stufe (33) vorgesehen ist.

4. Selbstzerstörende Sicherheitsspritze nach Anspruch 3, **dadurch gekennzeichnet, dass** eine Innenwand der Nadelhalterung (3) ferner mit einem Vorsprung (35) als Positionsgrenze zur Anordnung der Nadel (4) vorgesehen ist.

5. Selbstzerstörende Sicherheitsspritze nach Anspruch 1, **dadurch gekennzeichnet, dass** die Spritzenröhre (1) innen mit einem Flüssigkeitsstoppring (8) versehen ist, der auf der Nadelhalterung (3) muffenartig angeordnet ist, um die Nadelhalterung (3) festzuhalten und einen Spalt zwischen der Nadelhalterung (3) und der Innenwand des Spritzenzylinders (1) abzudichten, um ein Entweichen von Flüssigkeit zu verhindern.

6. Selbstzerstörende Sicherheitsspritze nach Anspruch 5, **dadurch gekennzeichnet, dass** eine vordere Fläche des Flüssigkeitsstopprings (8) eine Kammer mit der Innenwand der Spritzenröhre (1) als Raum bildet, in dem der Flüssigkeitsstoppring (8) nach vom gleiten und sich aus der Nadelhalterung (3) lösen kann.

7. Selbstzerstörende Sicherheitsspritze nach Anspruch 6, **dadurch gekennzeichnet, dass** der Umfang eines oberen Endes der Stößelstange (2) mit einem ringförmigen oberen Block (21) versehen ist, um den Flüssigkeitsstoppring (8) nach vorn zu schieben, so dass dieser nach vom gleiten kann, um sich aus der Nadelhalterung (3) zu lösen; und der ringförmige obere Block mit Flüssigkeitsdurchlasskanälen (22) versehen ist, so dass die in der Spritzenröhre (1) verbleibende Flüssigkeit durch die Flüssigkeitsdurchlasskanäle (22), wenn der ringförmige obere Block den Flüssigkeitsstoppring (8) tangiert und mit diesem in Eingriff tritt, in eine Kammer der Nadelhalterung (3) und der Nadel (4) strömt.

8. Selbstzerstörende Sicherheitsspritze nach Anspruch 5, **dadurch gekennzeichnet, dass** die Innenwand der Spritzenröhre (1) ferner mit einem konvexen Haltering (10) an einem hinteren Ende des Flüssigkeitsstopprings (8) versehen ist, um das Zurückweichen der Nadelhalterung (3) zu verhindern, wenn während der Injektion eine Kraft auf die Nadelhalterung (3) ausgeübt wird.

9. Selbstzerstörende Sicherheitsspritze nach einem der Ansprüche 2 und 6 bis 8, **dadurch gekennzeichnet, dass** der Kolben (5), der elastische Gummidichtring (63) und der Flüssigkeitsstoppring (8) aus synthetischen Gummimaterialien hergestellt sind.

10. Selbstzerstörende Sicherheitsspritze nach Anspruch 1, **dadurch gekennzeichnet, dass** sie ferner eine Nadelkanüle (11), die muffenartig auf einem vorderen Ende der Spritzenröhre (1) angeordnet ist, und einem oberen Stopfen (13) aufweist, der an einem Endabschnittsende der Stößelstange (2) vorgesehen ist.

## Revendications

1. Seringue de sécurité à usage unique auto-destructible exempte de fluide résiduel, comprenant un tube de seringue (1), une tige de poussée (2), une monture de canule (3) et une canule (4), la canule (4) étant montée sur la monture de canule (3), la monture de canule (3) et la tige de poussée (2) étant montées dans le tube de seringue (1), un piston (5) ajusté de manière coulissante sur une paroi intérieure du tube de seringue (1) étant en outre prévu à une extrémité avant de la tige de poussée (2), un moyen de rétraction automatique (6) étant monté dans une extrémité avant d'une chambre de la tige de poussée (2), le moyen de rétraction automatique (6) comportant une tige de rétraction (61) et un ressort de compression (62) entourant la tige de rétraction (61), la tige de rétraction (61) du moyen de rétraction automatique (6) et la monture de canule (3) étant pourvues chacune d'un élément de verrouillage par encliquetage (612 ; 332), respectif, les deux éléments de verrouillage par encliquetage (612 ; 332) formant un ajustement serré l'un avec l'autre pour accoupler la monture de canule (3) avec le moyen de rétraction automatique (6) ; **caractérisée en ce qu'**une rondelle élastique en caoutchouc (63) est montée à une extrémité terminale de la tige de rétraction (61) et une bague de limitation de position (64) est prévue sur une paroi intérieure de la tige de poussée (2), la rondelle élastique en caoutchouc (63) et la bague de limitation de position (64) forment un ajustement serré l'une avec l'autre pour fixer le moyen de rétraction automatique (6) dans l'extrémité avant de la chambre de la tige de poussée (2), chaque élément de verrouillage par encliquetage (612 ; 332) est prévu avec des canaux de passage fluidique (613 ; 333) de telle manière que le fluide restant à l'intérieur du tube de seringue (1) peut continuer à s'écouler par les canaux de passage fluidique (613 ; 333) et vers une chambre de la monture de canule (3) et la canule (4) quand les éléments de verrouillage par encliquetage (612 ; 332) forment un ajustement serré et ont prise l'un sur l'autre.

2. Seringue de sécurité auto-destructible selon la revendication 1, **caractérisée en ce qu'**une bague d'arrêt de fluide (8) est en outre prévue entre le moyen de rétraction automatique (6) et l'extrémité avant de la chambre de la tige de poussée (2) pour combler un interstice entre le moyen de rétraction automatique (6) et la chambre de la tige de poussée (2) afin d'empêcher une fuite de fluide.

3. Seringue de sécurité auto-destructible selon la revendication 1, **caractérisée en ce que** : la monture de canule (3) est prévue avec un premier étagement (31), un deuxième étagement (32) et un troisième étagement (33) ; le premier étagement (31) et un orifice de tube (14) à une extrémité supérieure du tube de seringue (1) s'ajustent l'un à l'autre pour empêcher une inclinaison de la canule (4) pendant l'injection ; un étagement à crochet (34) est prévu entre le deuxième étagement (32) et le troisième étagement (33) pour empêcher un recul de la monture de canule (3) quand une force est appliquée sur la monture de canule (3) pendant l'injection ; et l'élément de verrouillage par encliquetage (332) de la monture de canule (3) est prévu sur le troisième étagement (33).

4. Seringue de sécurité auto-destructible selon la revendication 3, **caractérisée en ce qu'**une paroi intérieure de la monture de canule (3) est en outre prévue avec une gorge (35) en tant que limite de position pour le montage de la canule (4).

5. Seringue de sécurité auto-destructible selon la revendication 1, **caractérisée en ce que** le tube de seringue (1) est prévu intérieurement avec une bague d'arrêt de fluide (8) entourant la monture de canule (3), pour fixer la monture de canule (3) et combler un interstice entre la monture de canule (3) et la paroi intérieure du tube de seringue (1) afin d'empêcher une fuite de fluide.

6. Seringue de sécurité auto-destructible selon la revendication 5, **caractérisée en ce qu'**une surface avant de la bague d'arrêt de fluide (8) forme une chambre avec la paroi intérieure du tube de seringue (1), en tant qu'espace permettant à la bague d'arrêt de fluide (8) de coulisser vers l'avant et de se désengager de la monture de canule (3).

7. Seringue de sécurité auto-destructible selon la revendication 6, **caractérisée en ce que** la circonférence d'une extrémité supérieure de la tige de poussée (2) est prévue avec un bloc supérieur annulaire (21) pour repousser la bague d'arrêt de fluide (8) vers l'avant, lui permettant ainsi de coulisser vers l'avant et de se désengager de la monture de canule (3) ; et **en ce que** le bloc supérieur annulaire (21) est pourvu de canaux de passage fluidique (22) permettant l'écoulement du fluide restant à l'intérieur du tube de seringue (1) par les canaux de passage fluidique (22) vers une chambre de la monture de canule (3) et la canule (4), quand le bloc supérieur annulaire (21) est tangent à, et en prise avec la bague d'arrêt de fluide (8).

8. Seringue de sécurité auto-destructible selon la revendication 5, **caractérisée en ce que** la paroi intérieure du tube de seringue (1) est en outre prévue avec une bague de retenue convexe (10) à une extrémité arrière de la bague d'arrêt de fluide (8) pour empêcher un recul de la monture de canule (3) quand une force est appliquée sur la monture de canule (3) pendant l'injection.

9. Seringue de sécurité auto-destructible selon l'une des revendications 2 et 6 à 8, **caractérisée en ce que** le piston (5), la rondelle élastique en caoutchouc (63) et la bague d'arrêt de fluide (8) sont en matériaux à base de caoutchouc synthétique.

10. Seringue de sécurité auto-destructible selon la revendication 1, **caractérisée en ce qu'**elle comprend en outre un cylindre de canule (11) entourant une extrémité avant du tube de seringue (1) et un bouchon d'extrémité (13) prévu à une extrémité terminale de la tige de poussée (2).
